# EUROPEAN PATENT SPECIFICATION

(11) **EP 1 302 169 B1**
(45) Date of publication and mention of the grant of the patent: **05.11.2008**
(21) Application number: 02257087.3
(22) Date of filing: 11.10.2002
(51) Int. Cl.: A61B 17/70

(54) **Spinal polyaxial cross connector**
Mehrachsiger Querverbinder für die Wirbelsäule
Dispositif polyaxial de liaison transversale pour le rachis

(30) Priority: 12.10.2001 US 328748 P
(43) Date of publication of application: 16.04.2003
(73) Proprietor: DePuy Spine, Inc., Raynham, MA 02767-0350 (US)
(72) Inventor: Burgess, Ian, Barrington, RI 02806 (US); Hawkins, Riley, Cumberland, RI 02864 (US); Bono, Frank, Rohoboth, MA 02169 (US); McCrea, Conor R., Quincy, MA 02745 (US); Ramsay, Christopher L., New Bedford, MA 02745 (US)
(74) Representative: Orr, Robert

(56) References cited:
- EP-A- 0 956 829
- WO-A-00/59387
- WO-A-01/24718
- FR-A- 2 795 622
- FR-A- 2 816 195
- PATENT ABSTRACTS OF JAPAN vol. 1999, no. 14, 22 December 1999 (1999-12-22) & JP 11 244299 A (ROBERT REED SHOKAI KK), 14 September 1999 (1999-09-14)

## Description

This invention relates generally to spinal instrumentation and more particularly to an apparatus for making connections between two spinal rods.

Typical spinal surgery employs screws anchored into adjoining vertebrae and longitudinal members between them so as to stabilize a position of the vertebrae with respect to each other. The longitudinal members may comprise plates or rods. Typically two such longitudinal members are employed, one on either side of the vertebrae. Stability is further enhanced through application of one or more transverse cross connectors connecting the two longitudinal members. A typical example is disclosed in US-5522816.

US-A-2002/0169448 (FR-A-2 795 622) discloses a cross connector for linking longitudinal members engaged to a spine as defined in the preamble of claim 1.

According to the invention as defined in claim 1, the invention provides a cross connector for linking longitudinal members engaged to a spine comprises a first connector for attaching to a first one of the longitudinal members, a second connector for attaching to a second one of the longitudinal members and a linkage between the first and second connectors. At least one polyaxial joint is located between the first and second connectors. The first connector comprises a first clamping member and a second clamping member, and a fastener adapted to hold the first clamping member and second clamping member tightly together whereby to grasp one of the longitudinal members. At least a portion of the at least one polyaxial joint is disposed between the first clamping member and the second clamping member, whereby when the fastener holds the first clamping member and the second clamping member together, said portion is squeezed to prevent rotation of the at least one polyaxial joint.

Either or both of the first and second connectors can comprise a polyaxial joint. Preferably, the second connector comprises a first clamping member and a second clamping member, and a fastener adapted to hold the first clamping member and second clamping member tightly together so as to grasp one of the longitudinal members. In one embodiment of the invention there is provided a second polyaxial joint, at least a portion of the second polyaxial joint is disposed between the first clamping member and the second clamping member of the second connector whereby when the fastener holds the first clamping member and second clamping member tightly together, that portion is squeezed to prevent rotation of the second polyaxial joint. Preferably, the first clamping member has a first surface adapted to engage one of the longitudinal members and the second clamping member has a second surface adapted to engage one of the longitudinal members with the fastener located between the portion of the polyaxial joint and the first and second surfaces.

Preferably, the polyaxial joint comprises a convex surface portion on the linkage and a bearing surface on one of the first and second connectors. The convex portion is preferably spherical.

Preferably, the linkage is curved whereby to arch over a patient's spine. It can comprise a first transverse member and a second transverse member connected in sliding relationship to each other whereby to alter a length of the linkage. Splines and grooves are preferably provided on the mating surfaces thereof to allow sliding and which fit together in an interference fit when compressed together whereby to prevent sliding of the first and second transverse members with respect to each other.

Preferably, the polyaxial joint comprises a curved surface connected to either the first connector or second connector and a mating surface thereto connected to the other of the first connector or second connector. The curved surface and mating surface are adapted for movement in three degrees of freedom over one another.

Preferably, the curved surface comprises a convex surface and the mating surface is a complementary concave surface, to fix the convex surface to the concave surface a first threaded aperture penetrates the convex surface, a second aperture is proved through a portion of the cross connector bearing the concave surface and a threaded connector passes through the second aperture and threads into the first aperture. Preferably, the second aperture is wider than the threaded connector.

Preferably, a camming member traps one of the longitudinal members into one of the connectors.

### Brief description of the drawings

FIG. 1 is a perspective view of a first embodiment of a cross connector according to a preferred embodiment of the present invention;
FIG. 1A is a sectional view taken along lines 1A--1A of FIG. 1;
FIG. 2 is a top plan view of the cross connector of FIG. 1;
FIG. 3 is a sectional view taken along lines 3--3 of FIG. 2;
FIG. 4 is a top plan view of a second embodiment of a cross connector according to a preferred embodiment of the present invention;
FIG. 5 is a section view taken along lines 5--5 of FIG. 4.

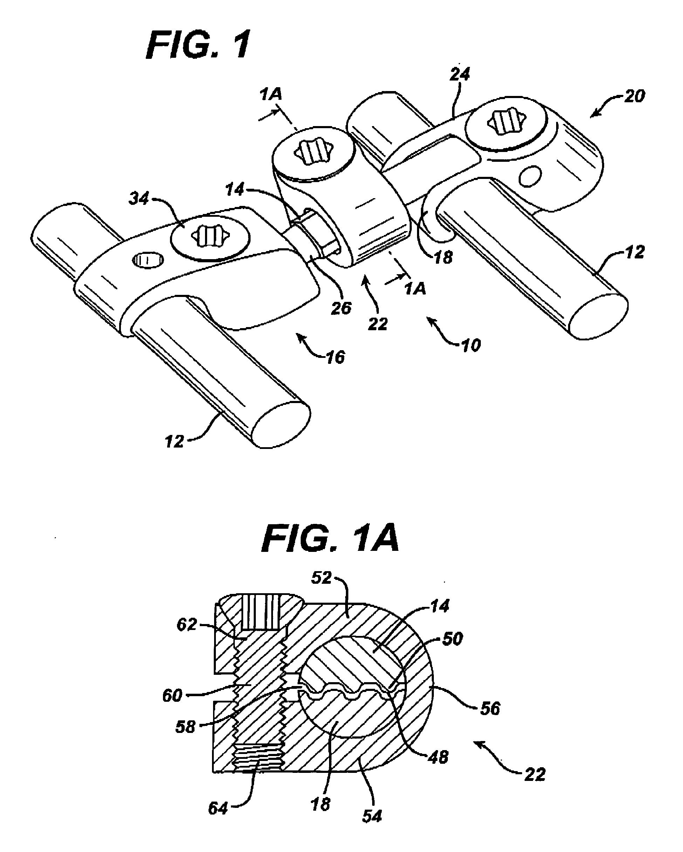

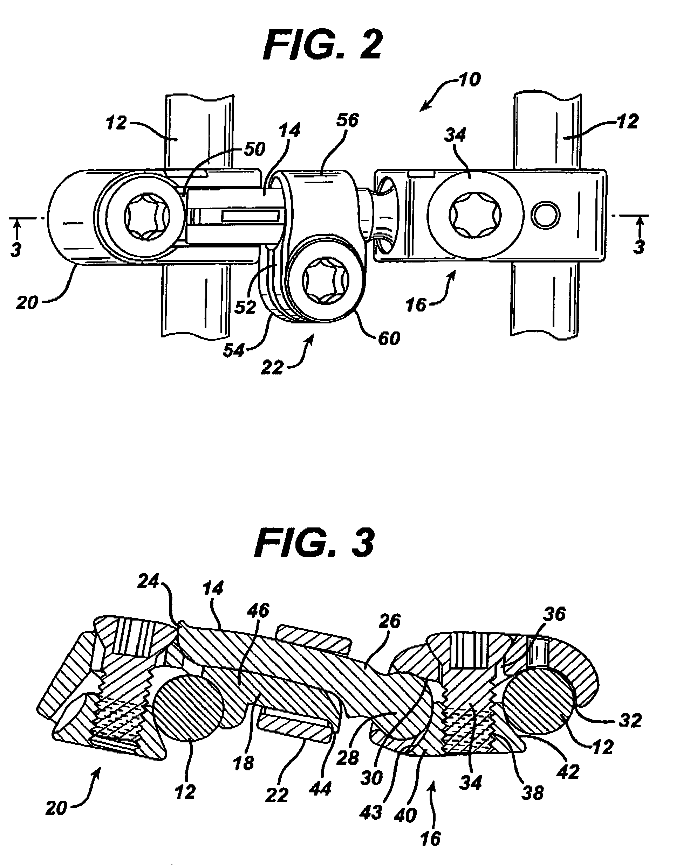

Referring to the drawings, Figures 1 to 3 illustrate a first embodiment of a cross connector 10 according to the present invention connected to two longitudinal members 12 as would be used to stabilize a spine during spinal surgery. The cross connector 10 comprises a first transverse member 14 having a clamp 16 for affixation to one of the longitudinal members 12, and a second transverse member 18 having a clamp 20 for affixation to the other longitudinal member 12. The first transverse member 14 and second transverse member 18 are locked together by a third clamp 22.

The first transverse member 14 arcs slightly from a proximal end 24 thereof to a distal end 26 thereof and terminates in a ball 28 at the distal end 26. The first clamp 16 comprises an inner curved surface 30 for receiving the ball 28 and a second curved surface 32 for receiving the longitudinal member 12. A first threaded connector 34 passes through an aperture 36 in the first clamp 16 between the first curved surface 30 and second curved surface 32 and engages a threaded aperture 38 in a flanged nut 40. The flanged nut 40 has a curved surface 42 for engaging longitudinal member 12 and an adjacent curved surface 43 for engaging the ball 28. Tightening the threaded connector 34 against the flanged nut 40 clamps the longitudinal member 12 between the flanged nut curved surface 42 and the curved surface 32 on the first clamp 16. It also clamps the ball 28 between the curved surface 42 on the flanged nut 40 and the curved surface 30 on the first clamp 16 thereby inhibiting rotation of the first clamp 16 about the ball 28. Thus the first clamp 16 clamps to the longitudinal member 12 and locks against the ball 28 by tightening a single screw, the threaded connector 34.

The second transverse member 18 arcs slightly from a proximal end 44 thereof to a distal end 46, terminating in the second clamp 20. The second transverse member 18 has a pair of grooves 48 on its upper surface between the proximal end 44 and distal end 46. Conversely, the first transverse member 14 has a pair of splines 50 on a lower surface between its proximal end 24 and distal end 26, which interconnect with the grooves 48 whereby to allow sliding transverse movement between the first transverse member 14 and second transverse member 18.

The third clamp 22 is a C-clamp design which wraps around the first transverse member 14 and second transverse member 18 to compress these two parts into clamping engagement. Accordingly, it comprises a pair of spaced apart arms 52 and 54 connected by an arcing portion 56 to form a transverse aperture 58 through them for receiving the first and second transverse members 14 and 18. A screw 60 penetrates a non-threaded aperture 62 on the first arm 52 and enters a threaded aperture 64 on the second arm 54 to tighten the third clamp 22 about the first and second transverse members 14 and 18.

The splines 50 and grooves 48 are formed with a taper lock so that as the clamp 22 tightens the splines 50 engage the grooves 48 with an interference fit. This greatly enhances the holding power of the clamp 22. The arc shape of the transverse members 14 and 18 also enhances the ability of the clamp 22 to resist slippage of the transverse members 14 and 18 as the forces tending to cause slippage will be translated into torque about the arc.

In use, the longitudinal members 12 are fixed to the spine in a traditional manner by means of screws as is known by those of skill in the art. The cross connector 10 connects the two longitudinal members 12 to form a more rigid support for the spine. The sliding movement of the first transverse member 14 with respect to the second transverse member 18 allows the space between the first clamp 16 and second clamp 20 to be adjusted to account for the inevitable variations in spacing of the longitudinal members 12 due to the anatomy of a particular patient. The ball 28 on the first clamp 16 allows polyaxial movement of the first clamp 16 with respect to the rest of the cross connector and can thereby account for longitudinal members 12 which may not be in perfect parallel alignment. It can also allow the surgeon to provide the lowest possible profile for the cross connector 10 by adjusting the distance of the cross connector 10 with respect to the spine by rotation of the first clamp 16 about the ball 28.

Of course, a similar ball joint arrangements could be provided in some other location between the first and second clamps 16 and 20, however, the particular arrangement of the ball 28 as shown inside the first clamped 16 allow a very few number of parts to achieve the clamping action and polyaxial movement.

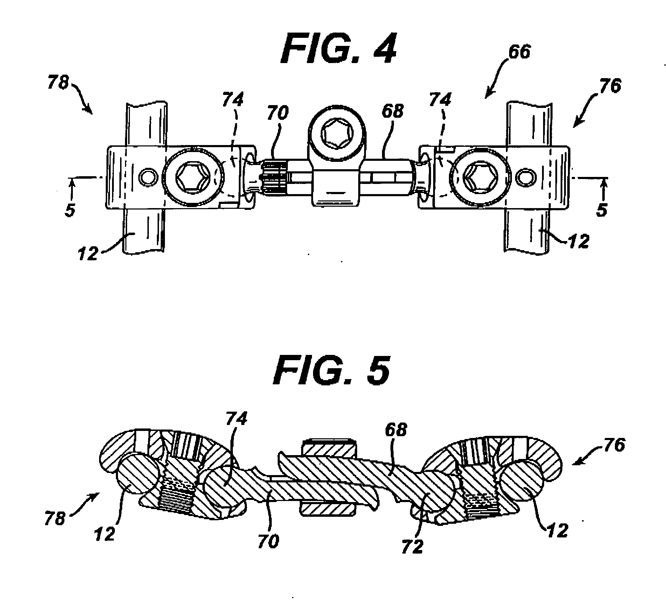

FIGS. 4 and 5 illustrate a cross connector 66 very similar to the cross connector 10 of the previous embodiment. However, the cross connector 66 has a ball joint construction on each clamp. Cross connector 66 comprises first and second transverse members 68 and 70 each terminating in balls 72 and 74, respectively. Affixed to each ball 72 and 74 is a first clamp 76 and second clamp 78, respectively, each of the same construction as the first clamp 16 of the prior embodiment. Accordingly, the cross connector 66 allows polyaxial rotation of the first and second clamps 76 and 78 and enhances the options of the surgeon in disposing this device within a patient's body.

While curved surfaces are shown for contact with the balls and longitudinal members, flat or angled surfaces may be used, as is known.

## Claims

1. A cross connector (10) adapted to link longitudinal members (12) engaged to a spine, the cross connector (10) comprising:
a first connector (16) adapted to attach to a first one of the longitudinal members (12),
a second connector (20) adapted to attach to a second one of the longitudinal members (12),
a linkage (14, 18) between the first and second connectors (16, 20), and
at least one polyaxial joint (28, 30, 43) located between the first and second connectors (16, 20),
**characterised in that** the first connector (16) comprises a first clamping member (16) and a second clamping member (40), and a fastener (34) adapted to hold the first clamping member (16) and second clamping member (40) tightly together so as to grasp one of the longitudinal members (12), at least a portion of the at least one polyaxial joint (28, 30, 43) being disposed between the first clamping member (16) and the second clamping member (40), whereby when the fastener (34) holds the first clamping member (16) and the second clamping member (40) together, said portion is squeezed to prevent rotation of the at least one polyaxial joint (28, 30, 43).

2. A cross connector (10) according to claim 1, in which one of the first and second connectors (16, 20) comprises the polyaxial joint (28, 30, 43).

3. A cross connector (10) according to claim 1, in which the second connector (78) comprises a first clamping member and a second clamping member, and a fastener adapted to hold the first clamping member and second clamping member tightly together so as to grasp one of the longitudinal members (12).

4. A cross connector (10) according to claim 3, further comprising a second polyaxial joint (74) in which at least a portion of the second polyaxial joint (74) is disposed between the first clamping member and the second clamping member of the second connector (78) whereby when the fastener holds the first clamping member and second clamping member tightly together, said portion is squeezed to prevent rotation of the second polyaxial joint (74).

5. A cross connector (10) according to claim 1, in which the first clamping member (16) has a first surface (32) adapted to engage one of the longitudinal members (12) and in which the second clamping member (40) has a second surface (42) adapted to engage one of the longitudinal members (12) and in which the fastener (34) is located between the at least a portion of the polyaxial joint (28, 30, 43) and the first and second surfaces (32, 34).

6. A cross connector (10) according to claim 1, in which the at least one polyaxial joint (28, 30, 43) comprises a convex surface portion (28) on the linkage (14) and a bearing surface (30, 43) on one of the first and second connectors (16, 20).

7. A cross connector (10) according to claim 6, in which the convex portion (28, 30, 43) is spherical.

8. A cross connector (10) according to claim 6, in which one of the first and second connectors (16, 20) comprises a first clamping member (16) and a second clamping member (40), and a fastener (34) adapted to hold the first clamping member (16) and second clamping (40) member tightly together whereby to grasp one of the longitudinal members (12) and in which the convex portion (28) is disposed between the first clamping member (16) and the second clamping member (40).

9. A cross connector (10) according to claim 1, in which the linkage (14, 18) is curved whereby to arch over a patient's spine.

10. A cross connector (10) according to claim 9, in which the linkage (14, 18) comprises a first transverse member (14) and a second transverse member (18) connected in sliding relationship to each other whereby to alter a length of the linkage (14, 18).

11. A cross connector (10) according to claim 10, in which one of the first and second transverse members (14, 18) bears at least one groove (40) and the other of the first and second transverse members (14, 18) bears at least one spline (50) slidably received within said groove (48) and in which the spline (50) fits within the groove (48) with an interference fit when compressed together whereby to prevent sliding of the first and second transverse members (14, 18) with respect to each other.

12. A cross connector (10) according to claim 1, in which the polyaxial joint (28, 30, 43) comprises a curved surface (30, 43) connected to one of the first connector (16) and second connector (20) and a mating surface (28) thereto connected to the other of the first connector (16) and second connector (20), the curved surface (30, 43) and mating surface (28) being adapted for movement in three degrees of freedom over one another.

13. A cross connector (10) according to claim 12, in which the curved surface (30, 43) comprises a convex surface (28) and the mating surface is a complementary concave surface.

14. A cross connector (10) according to claim 13, which includes a first threaded aperture penetrating the convex surface, a second aperture through a portion of the cross connector bearing the concave surface and a threaded connector passing through the second aperture and threading into the first aperture to fix the convex surface to the concave surface.

15. A cross connector (10) according to claim 14, in which the second aperture is wider than the threaded connector.

16. A cross connector (10) according to claim 1, in which one of the first and second connectors (16, 20) comprises a camming member which cams over one of the longitudinal members (12) trapping the longitudinal member (12) between the camming member and a surface in the connector.

## Patentansprüche

1. Querverbinder (10), der dazu ausgelegt ist, längs verlaufende Elemente (12) zu verbinden, die mit einer Wirbelsäure im Eingriff sind, wobei der Querverbinder (10) aufweist:
Ein erstes Anschlussstück (16), das dazu ausgelegt ist, an einem ersten der längs verlaufenden Elemente (12) befestigt zu werden,
ein zweites Anschlussstück (20), das dazu ausgelegt ist, an einem zweiten der längs verlaufenden Elemente (12) befestigt zu werden;
eine Verbindung (14, 18) zwischen dem ersten und dem zweiten Anschlussstück (16, 20) und wenigstens ein mehrachsiges Gelenk (28, 30, 43), das sich zwischen dem ersten und dem zweiten Anschlussstück (16, 20) befindet,
**dadurch gekennzeichnet, dass** das erste Anschlussstück (16) ein erstes Klemmelement (16) und ein zweites Klemmelement (40) und ein Befestigungselement (34) aufweist, das dazu ausgelegt ist, das erste Klemmelement (16) und das zweite Klemmelement (40) fest zusammenzuhalten, um so eines der längs verlaufenden Elemente (12) zu greifen, wobei wenigstens ein Teil des wenigstens einen mehrachsigen Gelenks (28, 30, 43) zwischen dem ersten Klemmelement (16) und dem zweiten Klemmelement (40) angeordnet ist, so dass, wenn das Befestigungselement (34) das erste Klemmelement (16) und das zweite Klemmelement (40) zusammenhält, der Teil gequetscht wird, um die Drehung des wenigstens einen mehrachsigen Gelenks (28, 30, 43) zu verhindern.

2. Querverbinder (10) nach Anspruch 1, bei dem eines, das erste oder das zweite Anschlussstück (16, 20) das mehrachsige Gelenk (28, 30, 43) aufweist.

3. Querverbinder (10) nach Anspruch 1, bei dem das zweite Anschlussstück (78) ein erstes Klemmelement und ein zweites Klemmelement und ein Befestigungselement, das dazu ausgelegt ist, das erste Klemmelement und das zweite Klemmelement fest zusammenzuhalten, um so eines der längs verlaufenden Elemente (12) zu greifen, aufweist.

4. Querverbinder (10) nach Anspruch 3, weiter mit einem zweiten mehrachsigen Gelenk (74), bei dem wenigstens ein Teil des zweiten mehrachsigen Gelenks (74) zwischen dem ersten Klemmelement und dem zweiten Klemmelement des zweiten Anschlussstücks (78) angeordnet ist, so dass, wenn das Befestigungselement das erste Klemmelement und das zweite Klemmelement fest zusammenhält, der Teil gequetscht wird, um die Drehung des zweiten mehraxialen Gelenks (74) zu verhindern.

5. Querverbinder (10) nach Anspruch 1, bei dem das erste Klemmelement (16) eine erste Fläche (32) hat, die dazu ausgelegt, an einem der längs verlaufenden Elemente (12) anzugreifen, und bei dem das zweite Klemmelement (40) eine zweite Fläche (42) hat, die dazu ausgelegt ist, an einem der längs verlaufenden Elemente (12) anzugreifen, und bei dem das Befestigungselement (34) sich zwischen dem wenigstens einen Teil des mehrachsigen Gelenks (28, 30, 43) und der ersten und der zweiten Fläche (32, 34) befindet.

6. Querverbinder (10) nach Anspruch 1, bei dem das wenigstens eine mehrachsige Gelenk (28, 30, 43) einen konvexen Flächenabschnitt (28) auf der Verbindung (14) und eine Lagerfläche (30, 43) auf einem, dem ersten oder dem zweiten Anschlussstück (16, 20), aufweist.

7. Querverbinder (10) nach Anspruch 6, bei dem der konvexe Teil (28, 30, 43) kugelförmig ist.

8. Querverbinder (10) nach Anspruch 6, bei dem eines, das erste oder das zweite Anschlussstück (16, 20) ein erstes Klemmelement (16) und ein zweites Klemmelement (40) und ein Befestigungselement (34) aufweist, das dazu ausgelegt ist, das erste Klemmelement (16) und das zweite Klemmelement (40) fest zusammenzuhalten, um so eines der längs verlaufenden Elemente (12) zu greifen, und bei dem der konvexe Teil (28) zwischen dem ersten Klemmelement (16) und dem zweiten Klemmelement (40) angeordnet ist.

9. Querverbinder (10) nach Anspruch 1, bei dem die Verbindung (14, 18) gekrümmt ist, so dass sie einen Bogen über der Wirbelsäule eines Patienten bildet.

10. Querverbinder (10) nach Anspruch 9, bei dem die Verbindung (14, 18) ein erstes quer verlaufendes Element (14) und ein zweites quer verlaufendes Element (18) aufweist, die miteinander verschieblich verbunden sind, um so eine Länge der Verbindung (14, 18) zu ändern.

11. Querverbinder (10) nach Anspruch 10, bei dem wenigstens eines, das erste oder das zweite quer verlaufende Element (14, 18), wenigstens eine Nut (40) trägt und andere aus erstem und zweitem quer verlaufenden Element (14, 18) wenigstens ein Profil (50) trägt, das verschieblich innerhalb der Nut (48) aufgenommen ist, und bei dem das Profil (50) in die Nut (48) mit einem Presssitz passt, wenn zusammengedrückt wird, so dass das Verschieben des ersten und des zweiten quer verlaufenden Elements (14, 18) in Bezug aufeinander verhindert wird.

12. Querverbinder (10) nach Anspruch 1, bei dem das mehrachsige Gelenk (28, 30, 43) eine gekrümmte Fläche (30, 43) aufweist, die mit einem, dem ersten Anschlussstück (16) oder dem zweiten Anschlussstück (20) verbunden ist, und eine dazu passende Fläche (28), die mit dem anderen aus erstem Anschlussstück (16) und zweitem Anschlussstück (20) verbunden ist, wobei die gekrümmte Fläche (30, 43) und die passende Fläche (28) für die Bewegung in drei Freiheitsgraden übereinander ausgelegt sind.

13. Querverbinder (10) nach Anspruch 12, bei dem die gekrümmte Fläche (30, 43) eine konvexe Fläche (28) aufweist und die passende Fläche eine komplementäre konkave Fläche ist.

14. Querverbinder (10) nach Anspruch 13, der eine erste Gewindeöffnung, die die konvexe Fläche durchdringt, eine zweite Öffnung durch einen Teil des Querverbinders, der die konkave Fläche trägt, und einen Gewindeverbinder, der durch die zweite Öffnung verläuft und in die erste Öffnung geschraubt wird, um die konvexe Fläche an der konkaven Fläche festzulegen, umfasst.

15. Querverbinder (10) nach Anspruch 14, bei dem die zweite Öffnung weiter ist als der Gewindeverbinder.

16. Querverbinder (10) nach Anspruch 1, bei dem einer, der erste oder der zweite Anschlussstück (16, 20), ein Kurvenelement aufweist, das über eines der längs verlaufenden Elemente (12) verläuft, wobei das längs verlaufende Element (12) zwischen dem Kurvenelement und einer Fläche in dem Anschlussstück eingefangen wird.

## Revendications

1. Dispositif de liaison transversale (10) adapté pour relier des éléments longitudinaux (12) en prise avec un rachis, le dispositif de liaison transversale (10), comprenant :
➢ un premier connecteur (16) adapté pour être fixé à un premier élément parmi les éléments longitudinaux (12),
➢ un deuxième connecteur (20) adapté pour être fixé à un deuxième élément parmi les éléments longitudinaux (12),
➢ une liaison (14, 18) entre le premier et le deuxième connecteur (16, 20), et
➢ au moins un joint poly-axial (28, 30, 43) positionné entre le premier et le deuxième connecteur (16, 20),
**caractérisé en ce que** le premier connecteur (16) comprend un premier élément de serrage (16) et un deuxième élément de serrage (40), et une pièce de fixation (34) adaptée pour retenir le premier élément de serrage (16) et le deuxième élément de serrage (40) serrés ensemble de sorte à saisir un des éléments longitudinaux (12), au moins une partie de l'au moins un joint poly-axial (28, 30, 43) étant disposée entre le premier élément de serrage (16) et le deuxième élément de serrage (40), moyennant quoi, quand la pièce de fixation (34) retient ensemble le premier élément de serrage (16) et le deuxième élément de serrage (40), ladite partie est serrée pour empêcher la rotation de l'au moins un joint poly-axial (28, 30, 43).

2. Dispositif de liaison transversale (10) selon la revendication 1, dans lequel un connecteur parmi les premier et deuxième connecteurs (16, 20) comprend le joint poly-axial (28, 30, 43).

3. Dispositif de liaison transversale (10) selon la revendication 1, dans lequel le deuxième connecteur (78) comprend un premier élément de serrage et un deuxième élément de serrage, et une pièce de fixation adaptée pour serrer ensemble le premier élément de serrage et le deuxième élément de serrage de sorte à saisir un des éléments longitudinaux (12).

4. Dispositif de liaison transversale (10) selon la revendication 3, comprenant en outre un deuxième joint poly-axial (74), dans lequel au moins une partie du deuxième joint poly-axial (74) est disposée entre le premier élément de serrage et le deuxième élément de serrage du deuxième connecteur (78), moyennant quoi quand la pièce de fixation serre ensemble le premier élément de serrage et le deuxième élément de serrage, ladite partie est serrée pour empêcher la rotation du au moins un joint poly-axial (74).

5. Dispositif de liaison transversale (10) selon la revendication 1, dans lequel le premier élément de serrage (16) comporte une première surface (32) adaptée pour engager un des éléments longitudinaux (12), et dans lequel le deuxième élément de serrage (40) a une deuxième surface (42) adaptée pour engager un des éléments longitudinaux (12), et dans lequel la pièce de fixation (34) est positionnée entre la au moins une partie du joint poly-axial (28, 30, 43) et les première et deuxième surfaces (32, 42).

6. Dispositif de liaison transversale (10) selon la revendication 1, dans lequel l'au moins un joint poly-axial (28, 30, 43) comprend une partie de surface convexe (28) sur la liaison (14) et une surface porteuse (30, 43) sur l'un des premier et deuxième connecteurs (16, 20).

7. Dispositif de liaison transversale (10) selon la revendication 6, dans lequel la partie convexe (28, 30, 43) est sphérique.

8. Dispositif de liaison transversale (10) selon la revendication 6, dans lequel l'un des premier et deuxième connecteurs (16, 20) comprend un premier élément de serrage (16) et un deuxième élément de serrage (40), et une pièce de fixation (34) adaptée pour serrer ensemble le premier élément de serrage (16) et le deuxième élément de serrage (40), pour saisir ainsi un des éléments longitudinaux (12), et dans lequel la partie convexe (28) est disposée entre le premier élément de serrage (16) et le deuxième élément de serrage (40).

9. Dispositif de liaison transversale (10) selon la revendication 1, dans lequel la liaison (14, 18) est incurvée, moyennant quoi elle peut s'étendre en arc au-dessus du rachis d'un patient.

10. Dispositif de liaison transversale (10) selon la revendication 9, dans lequel la liaison (14, 18) comprend un premier élément transversal (14) et un deuxième élément transversal (18) reliés l'un à l'autre de manière coulissante, moyennant quoi il est possible de modifier une longueur de la liaison (14, 18).

11. Dispositif de liaison transversale (10) selon la revendication 10, dans lequel l'un des premier et deuxième éléments transversaux (14, 18) présente au moins une rainure (40) et l'autre des premier et deuxième éléments transversaux (14, 18) présente au moins une languette (50) reçue en coulissement à l'intérieur de ladite rainure (48), et dans lequel la languette (50) s'adapte dans la rainure (48) par ajustement avec serrage suite à leur compression mutuelle, moyennant quoi le coulissement mutuel du premier et du deuxième élément transversal (14, 18) est évité.

12. Dispositif de liaison transversale (10) selon la revendication 1, dans lequel le joint poly-axial (28, 30, 43) comprend une surface incurvée (30, 43) reliée à l'un d'entre le premier connecteur (16) et le deuxième connecteur (20), ainsi qu'une surface (28) conjuguée à celle-ci reliée à l'autre d'entre le premier connecteur (16) et le deuxième connecteur (20), la surface incurvée (30, 43) et la surface conjuguée (28) étant adaptées pour se déplacer l'une sur l'autre par un mouvement à trois degrés de liberté.

13. Dispositif de liaison transversale (10), selon la revendication 12, dans lequel la surface incurvée (30, 43) comprend une surface convexe (28) et la surface conjuguée est une surface concave complémentaire.

14. Dispositif de liaison transversale (10) selon la revendication 13, ledit dispositif comprenant une première ouverture filetée pénétrant dans la surface convexe, une deuxième ouverture à travers une partie du dispositif de liaison transversale présentant la surface concave et un connecteur fileté traversant la deuxième ouverture et se vissant dans la première ouverture pour fixer la surface convexe sur la surface concave.

15. Dispositif de liaison transversale (10) selon la revendication 14, dans lequel la deuxième ouverture est plus large que le connecteur fileté.

16. Dispositif de liaison transversale (10) selon la revendication 1, dans lequel l'un d'entre le premier et le deuxième connecteur (16, 20) comprend un élément de came qui entre en contact avec un des éléments longitudinaux (12) piégeant l'élément longitudinal (12) entre l'élément de came et une surface dans le dispositif de liaison transversale.
